**Europäisches Patentamt**

**European Patent Office**

**Office européen des brevets**

(19)

(11) Numéro de publication : **0 200 610 B1**

(12) **FASCICULE DE BREVET EUROPÉEN**

(45) Date de publication du fascicule du brevet :
**07.09.88**

(51) Int. Cl.⁴ : **A 61 B   5/04,** A 61 B   5/10, A 61 B   5/22

(21) Numéro de dépôt : **86400739.8**

(22) Date de dépôt : **07.04.86**

(54) **Dispositif pour la détection, l'étude et la surveillance de maladies, notamment cardiaques, se traduisant par des manifestations électriquement enregistrables.**

(30) Priorité : **09.04.85 MA 20630**

(43) Date de publication de la demande :
**10.12.86 Bulletin 86/45**

(45) Mention de la délivrance du brevet :
**07.09.88 Bulletin 88/36**

(84) Etats contractants désignés :
**AT BE CH DE GB IT LI LU NL SE**

(56) Documents cités :
**DE-A- 2 822 343**
**DE-A- 2 822 343**
**FR-A- 2 402 439**
**US-A- 4 051 522**
**ELECTRONIQUE ET MICRO-ELECTRONIQUE INDUS-TRIELLES, no. 227, 1er novembre 1976, pages 36-39, Paris, FR; J.-P. WAYMEL: "L'électronique au service de l'électroencéphalographie"**
**JOURNAL OF THE ACOUSTICAL SOCIETY OF AME-RICA, vol. 70, no. 3, septembre 1981, pages 683-686, New York, US; B.C. SONIES et al.: "Ultrasonic visuali-zation of tongue motion during speech"**
**IDEM**

(73) Titulaire : **SA MAJESTE HASSAN II ROI DU MAROC
Palais Royal
Rabat (MA)**

(72) Inventeur : **SA MAJESTE HASSAN II ROI DU MAROC
Palais Royal
Rabat (MA)**

(74) Mandataire : **Polus, Camille et al
c/o Cabinet Lavoix 2, Place d'Estienne d'Orves
F-75441 Paris Cedex 09 (FR)**

## Description

La présente invention concerne un dispositif pour la détection, l'étude et la surveillance des maladies, notamment cardiaques qui se traduisent par des manifestations électriquement enregistrables.

On sait que de nombreuses maladies ou malaises, (par exemple, les maladies cardiaques ou coronariennes) sont détectables par des détecteurs fournissant des informations électriquement enregistables (par exemple l'électrocardiogramme du patient).

Mais on sait aussi que la détection de ces maladies, à leur stade le plus précoce, nécessite des enregistrements très prolongés et nécessite très souvent de connaître, à l'instant où les manifestations desdites maladies se font sentir, l'activité physique exacte des sujets surveillés.

Le brevet US. 4 051 522 décrit un dispositif d'observation et d'enregistrement de l'activité d'au moins un organe du corps d'un patient se traduisant par des manifestations électriquement enregistrables, en vue notamment de l'étude et de la surveillance de maladies cardiaques.

Ce dispositif comporte des moyens vidéo de prise de vue d'un patient, des moyens de détection et de transmission de signaux électriques traduisant l'activité de l'organe considéré, des moyens de combinaison des signaux vidéo correspondant à l'image du patient et des signaux électriques traduisant l'activité dudit organe, des moyens d'enregistrement desdits signaux combinés et des moyens d'affichage simultané des images relatives aux mouvements du patient et de celles des signaux électriques traduisant l'activité dudit organe.

Les moyens de détection et de transmission des signaux traduisant l'activité dudit organe comprennent, des capteurs fixés au corps du patient de façon à recevoir les informations relatives à l'activité de cet organe et connectés à un émetteur porté par le patient et alimenté par une source d'énergie électrique autonome, et un récepteur des signaux de l'émetteur, ledit récepteur étant connecté à une entrée d'un convertisseur de courbe vidéo dont une autre entrée est connectée à la sortie desdits moyens de prise de vue, ledit convertisseur de courbe vidéo étant destiné à permettre l'enregistrement et/ou l'observation synchronisés des images relatives aux évolutions du patient prises par les moyens de prise de vue et celle du diagramme en fonction du temps du fonctionnement de l'organe considéré, ledit récepteur étant en outre connecté à une entrée d'un fréquencemètre vidéo dont une autre entrée est connectée à la sortie desdits moyens de prise de vue, ledit fréquencemètre vidéo étant destiné à permettre l'enregistrement et/ou l'observation synchronisés des images relatives aux évolutions du patient prises par les moyens de prise de vue et les images d'informations chiffrées relatives au fonctionnement de l'organe considéré.

Le but de la présente invention est de mettre à la disposition des médecins et chercheurs médicaux, un dispositif du type précité permettant de détecter dans les meilleures conditions et en rassemblant les divers éléments nécessaires, les maladies qui peuvent se traduire par une manifestation électriquement enregistrable.

L'invention a donc pour objet un dispositif d'observation et d'enregistrement de l'activité d'au moins un organe du corps d'un patient se traduisant par des manifestations électriquement enregistrables, en vue notamment de l'étude et de la surveillance de maladies cardiaques, comportant des moyens vidéo de prise de vue des mouvements du patient, des moyens de détection et de transmission de signaux électriques traduisant l'activité de l'organe considéré, comprenant, comme connu en soi, des capteurs fixés au corps du patient de façon à recevoir les informations relatives à l'activité de cet organe et connectés à un émetteur de télémétrie porté par le patient et un récepteur de télémétrie, des moyens de conversion et de combinaison des signaux vidéo relatifs aux évolutions du patient prises par les moyens de prise de vue avec des images d'informations chiffrées relatives au fonctionnement de l'organe considéré et des images du diagramme en fonction du temps de signaux électriques traduisant l'activité dudit organe, des moyens d'enregistrement desdits signaux combinés et des moyens d'affichage simultané des images relatives aux mouvements du patient, des images d'informations chiffrées et des images du diagramme en fonction du temps des signaux électriques traduisant l'activité dudit organe, caractérisé en ce qu'un fréquencemètre vidéo destiné à permettre l'enregistrement et/ou l'observation synchronisés des images relatives aux évolutions du patient prises par les moyens de prise de vue et des images d'informations chiffrées relatives au fonctionnement de l'organe considéré comprend au moins une entrée destinée à recevoir les signaux électriques traduisant l'activité de l'organe du patient connectée à une sortie du récepteur, une autre entrée destinée à recevoir un signal vidéo et de synchronisation connectée via une horloge vidéo à la sortie des moyens de prise de vue et une sortie connectée à une entrée de signal composite d'un convertisseur de courbe vidéo destiné à permettre l'enregistrement et/ou l'observation synchronisés des images relatives aux évolutions du patient et celles du diagramme en fonction du temps de signaux électriques traduisant l'activité dudit organe et comprenant au moins une autre entrée destinée à recevoir les signaux électriques traduisant l'activité de l'organe du patient, connectée à la sortie du récepteur et une sortie connectée aux moyens d'enregistrement vidéo et aux moyens de visualisation.

Un second mode de réalisation du dispositif selon l'invention est caractérisé en ce qu'un découpeur d'image (28) assurant la répartition des diverses informations vidéo en vue de leur affichage dans des zones correspondantes des moyens (9) d'affichage comprend une première entrée connectée à la sortie d'un fréquencemètre vidéo, (6) recevant sur au moins une entrée (6a, b, c, d) connectée à la sortie du récepteur (3) des signaux électriques traduisant l'activité dudit organe, une deuxième entrée connectée à la sortie d'un convertisseur de courbe vidéo (7) recevant sur au moins une entrée (7a, b, c, d) connectée à la sortie du récepteur (3) des signaux électriques traduisant l'activité dudit organe, une troisième entrée connectée à la sortie des moyens (4) de prise de vue, une quatrième entrée connectée à la sortie d'une horloge vidéo (5) et une sortie connectée auxdits moyens (8) d'enregistrement vidéo et moyens (9) d'affichage vidéo en vue de l'enregistrement et/ou l'affichage simultanés des images provenant des moyens (4) de prise de vue, des images d'informations chiffrées provenant du fréquencemètre vidéo (6), des images du diagramme en fonction du temps provenant du convertisseur de courbe vidéo (7) et des images provenant de l'horloge vidéo (5) dans des zones indépendantes et correspondantes de l'image vidéo.

Si les moyens qui constituent le dispositif selon l'invention peuvent être matériellement reliés les uns aux autres, l'invention se révèle tout spécialement intéressante lorsque le sujet à étudier a une indépendance totale dans ses exercices et ses déplacements, ce qui implique le plus souvent qu'il convient de prévoir des moyens permettant la transmission à distance des informations recueillies notamment en ce qui concerne l'électrocardiogramme qui sera transmis vers un appareil récepteur et éventuellement enregistreur du type appareil de télévision et magnétoscope.

Il est clair également que, si dans certains cas, il apparaît nécessaire de recueillir simultanément, outre l'image de patient et son électrocardiogramme, des informations complémentaires, telles que température, état de la peau, etc. il est possible dans le cadre de l'invention de mettre sur le patient un détecteur approprié permettant de recueillir l'information souhaitée et de transmettre cette information à l'assembleur utilisé dans l'invention.

Comme moyens vidéo suiveurs, on utilise un système vidéomagnétoscopique constitué essentiellement d'une petite caméra ayant un système de stockage des images filmées. Cette caméra est montée sur un pied pivotant. l'orientation de cette caméra (sur le patient) est réalisée grâce à un émetteur fixé sur 1, patient qui commande à un ou plusieurs moteurs asservis, lesquels orientent constamment la caméra sur le patient émetteur. Cette caméra enregistrera sur une portion du film (par exemple une moitié de ce film) les déplacements et les gestes du patient.

Les moyens de détection des pulsations du coeur sont connus, on utilise le plus souvent deux ou trois électrodes fixées sur le thorax du patient qui recueillent les potentiels d'action cardiaque émis tout au ·long des battements ces électrodes sont reliées à un boîtier de petite taille porté à la ceinture du patient qui enregistre l'électrocardiogramme et le transmet à la caméra décrite ci-dessus, de façon que cette dernière enregistre à son tour ledit électrocardiogramme sur le même film qui enregistre simultanément l'activité du patient. Ce boîtier est de préférence totalement étanche car il est souvent souhaitable d'utiliser le dispositif de l'invention lorsque le patient effectue des mouvements de natation. Les progrès de l'électronique permettent aujourd'hui d'envisager la suppression dudit boîtier et l'émission directe vers la caméra, en vue de l'enregistrement, des informations recueillies par chaque électrode, informations qui formeront l'électrocardiogramme du patient.

L'assembleur est, selon l'invention, constitué par la caméra elle-même ou son film qui recueille simultanément les informations, image et électrocardiogramme ; ce film est projeté et il est alors possible d'apprécier simultanément la forme et l'intensité d'un mouvement et les modifications induites par ce mouvement sur l'électrocardiogramme.

L'invention sera mieux comprise à l'aide de la description qui va suivre, donnée uniquement à titre d'exemple et faite en se référant aux dessins annexés, sur lesquels :

— la Fig. 1 est un schéma synoptique d'un mode de réalisation préféré du dispositif d'observation et d'enregistrement suivant l'invention ;

— la Fig. 2 est un schéma plus détaillé du fréquencemètre vidéo entrant dans la construction du dispositif de la Fig. 1 ;

— la Fig. 3 est un schéma partiel montrant une variante du fréquencemètre vidéo de la Fig. 2 ;

— la Fig. 4 est un schéma détaillé du dispositif convertisseur de courbe vidéo entrant dans la construction du dispositif de la Fig. 1 ;

— la Fig. 5 est un schéma synoptique d'un autre mode de réalisation du dispositif d'observation et d'enregistrement de la Fig. 1 ; et

— la Fig. 5A montre le découpage de l'écran du dispositif d'affichage de la Fig. 5.

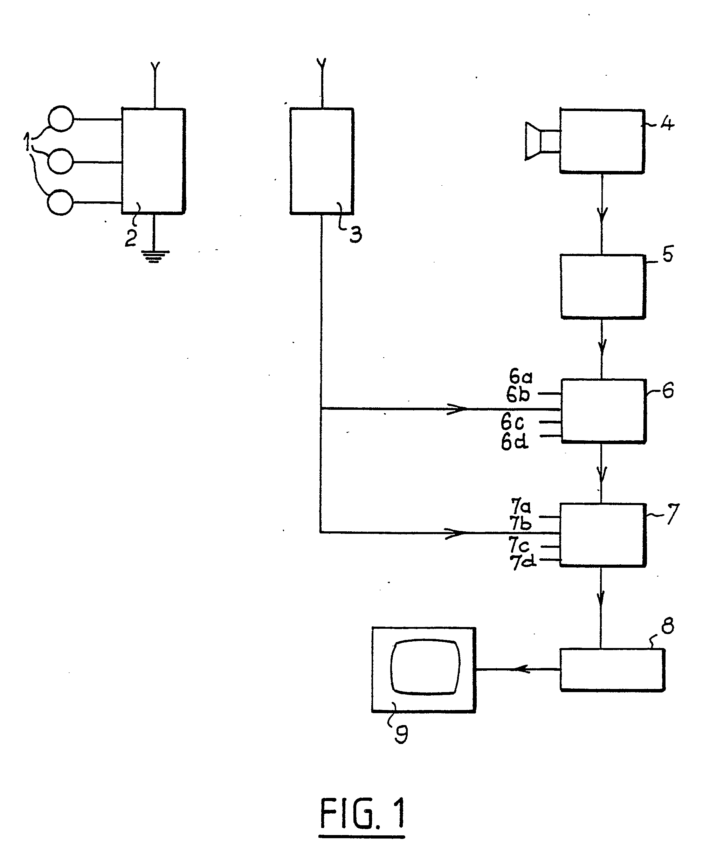

Le dispositif d'observation et d'enregistrement représenté à la Fig. 1 comporte trois capteurs 1 destinés à être fixés au thorax d'un patient en vue de détecter l'activité de son coeur et de délivrer des signaux électriques traduisant cette activité.

Les capteurs 1 sont connectés à un émetteur 2 alimenté par batterie et porté par le patient, par exemple à la ceinture ou sur le dos.

L'émetteur 2 est avantageusement placé dans un boîtier étanche, ce qui permet au patient qui en est muni d'évoluer dans une piscine.

Le dispositif suivant l'invention comporte en outre un récepteur 3 des signaux émis par l'émetteur 2 et une caméra de télévision 4 destinée à effectuer des prises de vue des évolutions du patient dont il y a lieu d'étudier le comportement du coeur.

La caméra 4 qui est avantageusement du type à focalisation automatique peut être portée par un opérateur chargé de suivre les évolutions du patient.

Elle peut être également montée sur un trépied convenablement disposé dans la zone d'évolution du patient et ses mouvements peuvent être commandés en azimut et en site par un dispositif classique formé par exemple d'un petit radar calé sur les signaux de l'un des capteurs 1 et commandant des moteurs électriques asservis correspondants.

La sortie de la caméra 4 est connectée à l'entrée d'une horloge vidéo 5 destinée à générer des signaux de synchronisation pour les signaux vidéo. L'horloge vidéo est en outre destinée à fournir des indications de date et d'heure de l'examen effectué. Elle joue également le rôle de chronomètre destiné à permettre d'effectuer des calculs sur des portions d'enregistrement d'un intérêt particulier pour le praticien.

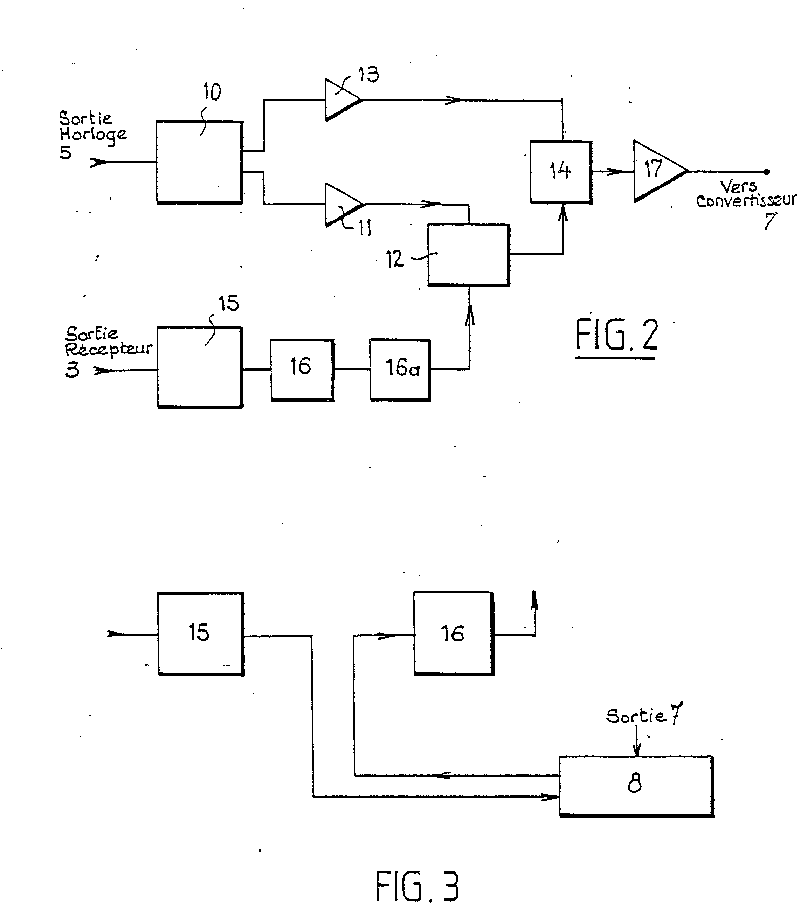

La sortie de l'horloge vidéo 5 est connectée à l'entrée d'un fréquencemètre vidéo 6 dont la constitution va être décrite en détail en référence à la Fig. 2.

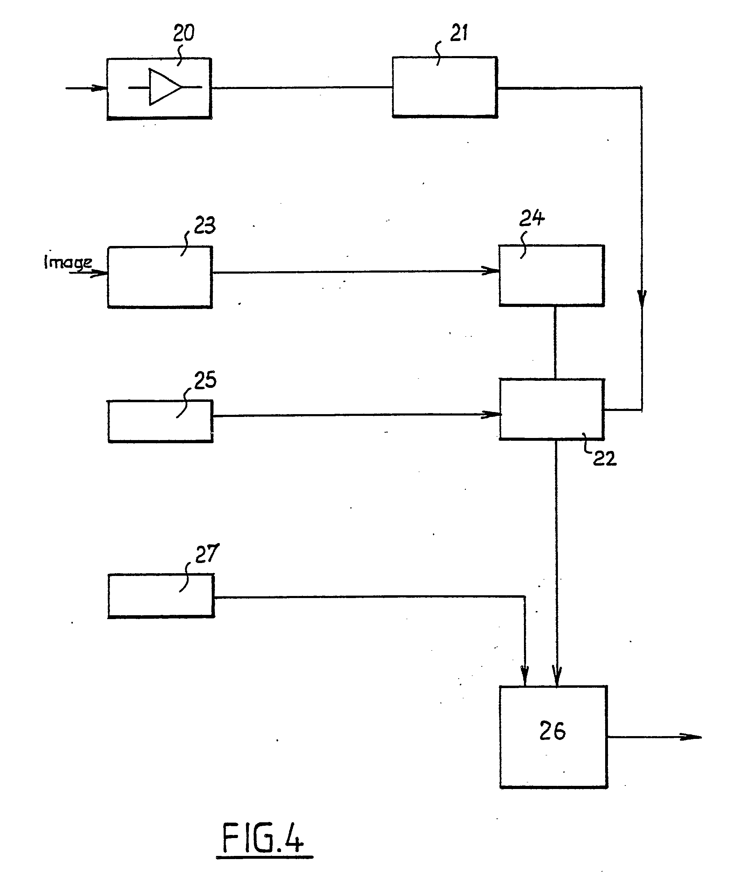

La sortie du fréquencemètre vidéo est connectée à l'entrée d'un convertisseur de courbe vidéo 7 qui sera décrit en détail en référence à la Fig. 4.

Le fréquencemètre vidéo 6 et le convertisseur de courbe vidéo 7 comportent chacun une entrée par laquelle ils sont connectés à la sortie du récepteur 3 des signaux d'activité cardiaque.

Ces deux dispositifs ou l'un d'entre eux au moins peuvent comporter des entrées supplémentaires permettant d'associer au signal vidéo relatif à l'image du patient, des informations relatives à l'activité d'autres organes de celui-ci ou encore à des paramètres de l'environnement dans lequel il évolue.

Ces entrées indiquées en 6a à 6d et 7a à 7d à la Fig. 1 peuvent également recevoir les signaux cardiaques de plusieurs patients ou encore d'athlètes dont on souhaite observer les réactions à l'effort.

La sortie du convertisseur de courbe vidéo 7 est connectée à l'entrée de la piste vidéo d'un magnétoscope 8 dont la sortie de lecture est connectée a un dispositif d'affichage à écran 9.

Bien entendu, il est également possible de connecter le dispositif d'affichage 9 directement à la sortie du convertisseur de courbe vidéo 7.

Dans ce cas, on ne disposera que d'informations en temps réel sur l'activité du patient et sur les réactions du ou des organes étudiés de celui-ci.

Le fréquencemètre vidéo représenté à la Fig. 2, est destiné à permettre l'affichage simultanément avec celui de l'image des évolutions du patient d'informations chiffrées telles que par exemple, la cadence du pouls, la tension artérielle, la température ou autres.

Il comprend principalement un circuit 10 destiné à assurer la séparation des signaux vidéo et de synchronisation qui composent le signal de sortie de l'horloge vidéo 5 (Fig. 1).

La sortie de signal vidéo du circuit est connectée par l'intermédiaire d'un amplificateur 11 à une entrée d'un circuit mélangeur 12.

La sortie de signal de synchronisation du circuit 10 est connectée par l'intermédiaire d'un amplificateur 13 à une entrée d'un circuit 14 de reconstitution du signal composite.

Le fréquencemètre vidéo comporte en outre une entrée pour le signal de sortie du récepteur 3, des signaux traduisant l'activité de l'organe à étudier.

Cette entrée est celle d'un convertisseur 15 de signaux analogiques en signaux numériques TTL comportant en outre un circuit de comptage des signaux reçus (non représentés).

La sortie du convertisseur 15 est connectée à l'entrée d'un circuit 16 de codage vidéo en composantes R, V, B du signal en couleur à obtenir.

La sortie du circuit 16 est connectée à l'entrée d'un circuit 16a de décodage dont la sortie est reliée à son tour à une autre entrée du circuit mélangeur 12.

La sortie du circuit mélangeur est reliée à une seconde entrée du circuit 14 de reconstitution du signal composite.

La sortie du circuit 14 est connectée par l'intermédiaire d'un amplificateur 17 à l'entrée de signal composite du convertisseur de courbe vidéo 7 (Fig. 1).

Selon une variante représentée à la Fig. 3, le convertisseur analogique-numérique 15 est connecté à l'entrée d'enregistrement d'une piste audio du magnétoscope 8, la sortie lecture de la piste audio de ce dernier étant reliée à l'entrée du circuit de codage vidéo.

Dans ce cas, la piste audio du magnétoscope 8 constitue une mémoire des signaux d'activité de l'organe surveillé.

La lecture de ces signaux pourra être faite en temps différé, en synchronisme avec les images correspondantes du patient.

Le convertisseur de courbe vidéo représenté à la Fig. 4 comporte une entrée analogique constituée par l'entrée d'un amplificateur 20 des signaux de sortie du récepteur 3. La sortie de l'amplificateur est connectée à l'entrée d'un convertisseur analogique-numérique 21 dont la sortie est reliée à l'entrée d'un décodeur-comparateur 22.

Le convertisseur de courbe vidéo comporte en outre une entrée vidéo connectée à la sortie du fréquencemètre vidéo 6 (Fig. 1).

Cette entrée vidéo est l'entrée d'un séparateur 23 dont la sortie est connectée à l'entrée d'un codeur vidéo 24.

Le séparateur 23 a pour fonction d'éliminer le signal de synchronisation du signal composite d'entrée.

Un générateur de signaux de synchronisation local 25 a sa sortie connectée à l'entrée de synchronisation du comparateur-décodeur 22.

La sortie du comparateur-décodeur 22 est connectée à une entrée d'un mélangeur de signaux 26 qui est en outre connecté par une autre de ses entrées à la sortie d'un générateur 27 de signaux d'intervalles de temps, par exemple d'une seconde.

La sortie du mélangeur 26 est connectée à l'entrée correspondante du magnétoscope 8 et constitue donc la sortie du convertisseur de courbes vidéo 7.

A titre de variante, la sortie de l'amplificateur 20 peut être connectée directement à une entrée d'enregistrement sur une piste audio du magnétoscope 8, la sortie de lecture correspondante de celui-ci étant alors reliée à l'entrée du convertisseur analogique-numérique 21. Un tel agencement permet un enregistrement direct des signaux traduisant l'activité de l'organe surveillé et une lecture différée de ces signaux en synchronisme avec les images correspondantes montrant les évolutions du patient.

Le dispositif qui vient d'être décrit fonctionne de la façon suivante.

Le patient qui porte sur lui les capteurs 1 convenablement disposés par rapport à l'organe à surveiller, par exemple le coeur, ainsi que l'émetteur 2, évolue dans une zone se trouvant dans le champ de la caméra vidéo 4. Cette zone peut être par exemple une piscine dans laquelle le patient se livre à des exercices de natation.

La caméra qui est soit manipulée par un opérateur, soit associée à un support avec des moyens de pointage automatique sur le patient, effectue des prises de vue des évolutions de celui-ci.

Simultanément, les capteurs 1 transmettent à l'émetteur 2, des signaux à basse fréquence correspondant à l'activité'de l'organe surveillé.

L'émetteur 2 transmet ces signaux au récepteur 3.

Les signaux de sortie de la caméra vidéo 4 sont appliqués à l'entrée de l'horloge vidéo 5 qui délivre à sa sortie un signal composite. Ce signal composite comporte des informations vidéo de synchronisation et chronométriques, ces dernières permettant au praticien de repérer des intervalles de temps remarquables dans l'enregistrement effectué en vue de procéder à des analyses détaillées des signaux enregistrés pendant ces intervalles. Le signal composite est appliqué à l'entrée du fréquencemètre vidéo 6 qui reçoit également les signaux de sortie du récepteur 3.

Dans le fréquencemètre vidéo 6, le signal composite provenant de l'horloge vidéo 5 est séparé en un signal vidéo et un signal de synchronisation. Le signal vidéo amplifié par l'amplificateur 11 est appliqué au circuit mélangeur 12.

Le signal de sortie du récepteur 3 est converti en numérique et compté de sorte que le signal numérique de sortie du convertisseur 15 correspond à la valeur instantanée du nombre de pulsations du coeur par unité de temps.

Le signal de sortie du convertisseur 15 est converti en signal analogique vidéo dans le convertisseur numérique-analogique 16, décodé dans le circuit 16a et appliqué au circuit mélangeur 12.

Celui-ci délivre à sa sortie un signal résultant de la superposition des signaux vidéo en provenance de la caméra et résultant de la conversion des signaux de sortie du récepteur 3 correspondant à l'activité de l'organe surveillé.

Ce signal de sortie est appliqué à une entrée du circuit 14 de reconstitution d'un signal composite qui reçoit à son autre entrée le signal de synchronisation à partir du séparateur 10.

Le signal de sortie du circuit 14 constitue après amplification, le signal destiné à être transmis au convertisseur de courbe vidéo 7.

Ce signal contient les informations chiffrées relatives au rythme cardiaque du patient destinées à être enregistrées par le magnétoscope 8 et à apparaître en clair sur l'écran d'affichage 9.

Il est appliqué à l'entrée du circuit séparateur 23 dont le signal de sortie ne comporte que le signal vidéo seul.

Ce signal est à son tour appliqué à l'entrée du codeur vidéo 24.

Le signal de sortie dudit codeur est appliqué à l'entrée correspondante du comparateur-décodeur 22.

Le signal de sortie de l'émetteur 3 (Fig.1) est appliqué à l'entrée de l'amplificateur 20.

Après amplification, ce signal est converti en signal numérique TTL et appliqué à l'entrée correspondante du comparateur décodeur 22.

Le signal de sortie du comparateur 22 contient à la fois les informations sous forme vidéo, de l'image du patient au cours de son activité, de la valeur chiffrée du rythme cardiaque et du diagramme en fonction du temps du fonctionnement du coeur.

Ce signal est rendu composite au moyen du signal de synchronisation provenant du générateur de signaux de synchronisation 25. Il est appliqué au mélangeur de signaux 26 qui reçoit en outre un signal d'intervalle de temps généré par le générateur 27.

Le mélangeur 26 assure une répartition des signaux de diverses natures contenus dans le signal vidéo et son signal de sortie est appliqué au magnétoscope 8 en vue d'être enregistré, puis au dispositif d'affichage 9. Sur l'écran de ce dernier apparaissent simultanément l'image du patient en mouvement, la valeur chiffrée instantanée de son rythme cardiaque, la date et l'heure à laquelle l'enregistrement a lieu ainsi que le diagramme temporel de l'activité cardiaque du patient rapporté à une échelle de temps de 1s apparaissant en abcisse de ce diagramme.

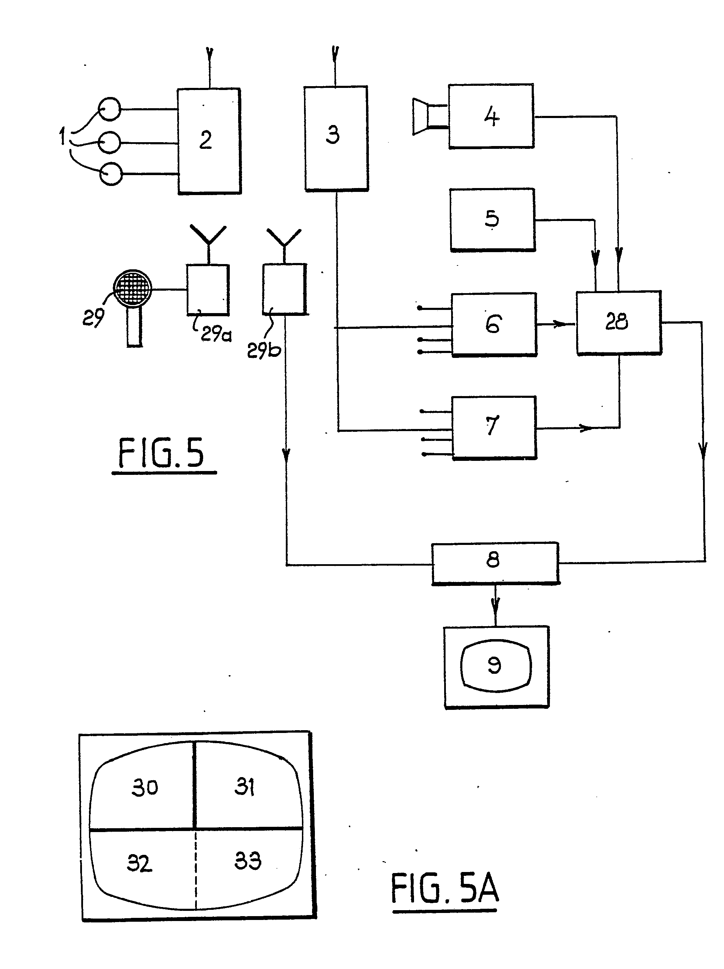

A la Fig. 5, on a représenté un autre mode de réalisation du dispositif suivant l'invention.

Sur cette figure, les éléments du dispositif identiques à ceux des dispositifs de la Fig.1 sont désignés par les mêmes numéros de référence.

Ce dispositif comporte des capteurs 1 connectés à un émetteur 2 à alimentation autonome porté par le patient. Un récepteur 3 des signaux de l'émetteur 2 est connecté a un fréquencemètre vidéo 6 et à un convertisseur de courbe vidéo 7.

Le dispositif comporte en outre une caméra vidéo 4 et une horloge vidéo 5.

La caméra 4, l'horloge 5, le fréquencemètre vidéo 6 et le convertisseur de courbe vidéo 7 sont connectés à des entrées correspondantes d'un circuit découpeur d'image 28.

Le découpeur 28 assure la répartition des diverses informations vidéo contenues dans les signaux de sortie des circuits 6 et 7 en vue de leur affichage dans des zones correspondantes de l'écran du dispositif d'affichage 7 associé du magnétoscope 8, auquel est connectée la sortie du circuit découpeur 28.

Le dispositif de la Fig. 5 comporte enfin un microphone 29 que le patient peut porter sur lui pour faire des commentaires sur les sensations qu'il éprouve au cours de l'examen.

Le microphone est connecté à un émetteur 29a associé à un récepteur audio 29b connecté à une piste d'enregistrement audio du magnétoscope 8 qui enregistre les informations données par le patient simultanément avec les images de son activité.

Lors de la lecture, ces informations peuvent être utiles pour compléter l'analyse du praticien.

Bien entendu, un tel microphone associé aux moyens de transmission correspondants peut également être associé au dispositif de la Fig.1.

La répartition assurée par le découpeur 28 est représentée à la Fig. 5A qui schématise la surface de l'écran du dispositif d'affichage 9.

La partie supérieure gauche 30 de l'écran est réservée à l'affichage de l'image du patient, la partie supérieure droite 31 est destinée à l'affichage des valeurs chiffrées du rythme cardiaque, de l'heure et d'autres informations utiles provenant éventuellement d'autres entrées du fréquencemètre vidéo 6 qui sont alors connectées à des dispositifs d'acquisition de données relatives à ces autres informations chiffrées.

Les parties inférieures 32, 33 sont prévues pour l'affichage du diagramme en fonction du temps de l'activité du coeur.

Dans les modes de réalisation qui viennent d'être décrits, les capteurs 1 sont connectés à un émetteur commun 2. On peut cependant envisager d'associer un émetteur à chaque capteur, ce qui permettrait moyennant une miniaturisation de ces circuits, d'éviter au patient le port d'un émetteur relativement encombrant.

Les modes de réalisation qui viennent d'être décrits sont considérés comme étant appliqués à l'observation et à l'enregistrement de phénomènes cardiologiques en vue de l'étude et de la surveillance de maladies cardiaques.

On comprendra cependant que ce dispositif peut également être appliqué à l'étude de maladies, d'autres organes que le coeur, à condition que l'activité de l'organe concerné puisse être traduite en signaux électriques enregistrables.

L'invention peut également s'appliquer à l'étude du comportement de sportifs de haut niveau en tenant compte de facteurs de l'environnement dans lequel ils évoluent.

Le dispositif suivant l'invention est conçu pour une miniaturisation poussée.

Il est par exemple possible d'intégrer à la caméra, le récepteur 3, l'horloge 5, le fréquencemètre vidéo 6 et le convertisseur vidéo 7.

**Revendications**

1. Dispositif d'observation et d'enregistrement de l'activité d'au moins un organe du corps d'un patient se traduisant par des manifestations électriquement enregistrables, en vue notamment de l'étude et de la surveillance de maladies cardiaques, comportant des moyens vidéo (4) de prise de vue des mouvements du patient, des moyens (1, 2, 3) de détection et de transmission de signaux électriques traduisant l'activité de l'organe considéré, comprenant, comme connu en soi, des capteurs (1) fixés au corps du patient de façon à recevoir les informations relatives à l'activité de cet organe et connectés à un émetteur de télémétrie (2) porté par le patient et un récepteur de télémétrie (3), des moyens (6, 7) de conversion et de combinaison des signaux vidéo relatifs aux évolutions du patient prises par les moyens (4) de prise de vue avec des images d'informations chiffrées relatives au fonctionnement de l'organe considéré et des images du diagramme en fonction du temps de signaux électriques traduisant l'activité dudit organe, des moyens (8) d'enregistrement desdits signaux combinés et des moyens (9) d'affichage simultané des images relatives aux mouvements du patient, des images d'informations chiffrées et des images du diagramme en fonction du temps des signaux électriques traduisant l'activité dudit organe, caractérisé en ce qu'un fréquencemètre vidéo (6) destiné à permettre l'enregistrement et/ou l'observation synchronisés des images relatives aux évolutions du patient prises par les moyens (4) de prise de vue et des images d'informations chiffrées relatives au fonctionnement de l'organe considéré comprend au moins une entrée (6a, b, c, d) destinée à recevoir les signaux électriques traduisant l'activité de l'organe du patient connectée à une sortie du récepteur (3), une autre entrée destinée à recevoir un signal vidéo et de synchronisation connectée via une horloge vidéo (5) à la sortie des moyens de prise de vue (4) et une sortie connectée à une entrée de signal composite d'un convertisseur de courbe vidéo (7) destiné à permettre l'enregistrement et/ou l'observation synchronisés des images relatives aux évolutions du patient et celles du diagramme en fonction du temps de signaux électriques traduisant l'activité dudit organe et comprenant au moins une autre entrée (7a, b, c, d) destinée à recevoir les signaux électriques traduisant l'activité de l'organe du patient connectée à la sortie du récepteur (3) et une sortie connectée aux moyens (8) d'enregistrement vidéo et aux moyens (9) de visualisation.

2. Dispositif d'observation et d'enregistrement de l'activité d'au moins un organe du corps d'un patient se traduisant par des manifestations électriquement enregistrables, en vue notamment de l'étude et de la surveillance de maladies cardiaques, comportant des moyens vidéo (4) de prise de vue des mouvements du patient, des moyens (1, 2, 3) de détection et de transmission de signaux électriques traduisant l'activité de l'organe considéré, comprenant, comme connu en soi, des capteurs (1) fixés au corps du patient de façon à recevoir les informations relatives à l'activité de cet organe et connectés à un émetteur de télémétrie (2) porté par le patient et un récepteur de télémétrie (3), des moyens (6, 7) de conversion et de combinaison des signaux vidéo relatifs aux évolutions du patient prises par les moyens (4) de prise de vue avec des images d'informations chiffrées relatives au fonctionnement de l'organe considéré et des images du diagramme en fonction du temps de signaux électriques traduisant l'activité dudit organe, des moyens (8) d'enregistrement desdits signaux combinés et des moyens (9) d'affichage simultané des images relatives aux mouvements du patient, des images d'informations chiffrées et des images du diagramme en fonction du temps des signaux électriques traduisant l'activité dudit organe, caractérisé en ce qu'un découpeur d'image (28) assurant la répartition des diverses informations vidéo en vue de leur affichage dans des zones correspondantes des moyens (9) d'affichage comprend une première entrée connectée à la sortie d'un fréquencemètre vidéo (6) recevant sur au moins une entrée (6a, b, c, d) connectée à la sortie du récepteur (3) des signaux électriques traduisant l'activité dudit organe, une deuxième entrée connectée à la sortie d'un convertisseur de courbe vidéo (7) recevant sur au moins une entrée (7a, b, c, d) connectée à la sortie du récepteur (3) des signaux électriques traduisant l'activité dudit organe, une troisième entrée connectée à la sortie des moyens (4) de prise de vue, une quatrième entrée connectée à la sortie d'une horloge vidéo (6) et une sortie connectée auxdits moyens (8) d'enregistrement vidéo et moyens (9) d'affichage vidéo en vue de l'enregistrement et/ou l'affichage simultanés des images provenant des moyens (4) de prise de vue, des images d'informations chiffrées provenant du fréquencemètre vidéo (6), des images du diagramme en fonction du temps provenant du convertisseur de courbe vidéo (7) et des images provenant de l'horloge vidéo (5) dans des zones indépendantes et correspondantes de l'image vidéo.

3. Dispositif suivant l'une quelconque des revendications 1 et 2, caractérisé en ce que le convertisseur de courbe vidéo (7) comporte un convertisseur analogique-numérique (21) des signaux en provenance dudit récepteur (3), ledit convertisseur analogique-numérique étant connecté à une entrée d'un décodeur-comparateur (22) dont une autre entrée est connectée à la sortie d'un codeur vidéo (24) recevant à son entrée les signaux vidéo de sortie d'un circuit séparateur (23) des signaux provenant desdits moyens de prise de vue.

4. Dispositif suivant l'une des revendications 1 et 2, caractérisé en ce que le fréquencemètre vidéo (6) comporte un circuit mélangeur (12) dont une entrée est connectée à une sortie d'un circuit séparateur (10) des signaux provenant desdits moyens de prise de vue (4), une autre entrée dudit circuit mélangeur (12) étant reliée à des moyens (15, 16, 16a) de conversion des signaux de sortie dudit récepteur (3) en signaux vidéo.

5. Dispositif suivant la revendication 4, caractérisé en ce que lesdits moyens de conversion des signaux de sortie du récepteur (3) en signaux vidéo comportent un convertisseur analogique-numérique (15) dont l'entrée est connectée au récepteur (3) et dont la sortie est connectée à un circuit de codage vidéo (16), la sortie de ce dernier étant connectée à l'entrée correspondante du circuit mélangeur (12) par l'intermédiaire d'un circuit de décodage (16a).

6. Dispositif suivant la revendication 5, caractérisé en ce que la sortie du convertisseur analogique-numérique (13) est connectée à l'entrée d'enregistrement d'une piste audio desdits moyens d'enregistrement vidéo (8), la sortie de lecture correspondante de ces derniers étant reliée à l'entrée dudit circuit de codage vidéo (16).

7. Dispositif suivant la revendication 3, caractérisé en ce que l'entrée du convertisseur de courbe vidéo (7) destinée à recevoir les signaux dudit récepteur est connectée à une entrée d'enregistrement sur une piste audio desdits moyens d'enregistrement vidéo (8), la sortie correspondante de ces derniers étant reliée à l'entrée du convertisseur analogique-numérique (21).

8. Dispositif suivant l'une quelconque des revendications précédentes, caractérisé en ce qu'il comporte en outre un microphone (29) connecté à un émetteur (29a) porté par le patient et un récepteur (29b) des signaux dudit émetteur (29a) associé au microphone, ledit récepteur (29b) étant connecté à une entrée d'enregistrement sur piste audio desdits moyens d'enregistrement.

9. Dispositif suivant l'une des revendications 1 à 8, caractérisé en ce que ledit fréquencemètre vidéo (5) et/ou ledit convertisseur de courbe vidéo (7) comportent plusieurs entrées (6a à 6d ; 7a à 7d) similaires à l'entrée connectée audit récepteur (3) et destinées à recevoir des signaux relatifs à l'activité d'autres organes du patient ou d'organes similaires de plusieurs patients et/ou à des paramètres de l'environnement dans lequel évoluent le ou les patients.

10. Dispositif suivant l'une quelconque des revendications 1 à 9, caractérisé en ce que lesdits moyens de prise de vue (4) sont constitués par une caméra disposée sur un support dans la zone d'évolution du patient, et en ce qu'il est prévu des moyens d'orientation automatique de la caméra sur le patient comprenant des moteurs électriques asservis de déplacement de la caméra en azimut et en site, commandés par un dispositif détecteur des signaux de l'un des capteurs (1) portés par le patient.

**Claims**

1. A system for observing and recording the activity of at least one organ of a patient's body represented by electrically recordable phenomena, more particularly for the study and monitoring of heart diseases, comprising : video means (4) for photographing the patient's movements ; means (1, 2, 3,) for detecting and transmitting electric signals representing the activity of the organ in question and comprising in known manner : sensors (1) which are attached to the patient's body so as to receive information relating to the activity of such organ and are connected to a telemetry transmitter (2) worn by the patient and a telemetry receiver (3) ; means (6, 7) for converting and combining video signals relating to the patient's movements photographed by the photographing means (4) with images of digital information relating to the functioning of the organ in question and images of the time dependency diagram of electric signals representing the activity of such organs ; means (8) for recording such combined signals, and means (9) for the simultaneous display of the images relating to the patient's movements, images of digital information and images of the time dependency diagram of the electric signals representing the activity of such organ, characterized in that a video frequency meter (6) adapted to allow the synchronized recording and/or observation of the images relating to the patient's movements photographed by the photographing means (4) and the images of digital information relating to the functioning of the organ in question comprises : at least one input (6a, b, c, d) adapted to receive the electric signals representing the activity of the patient's organ and connected to an output of the receiver (3) ; another input adapted to receive a video and synchronization signal connected via a video clock (5) to the output of the photographing means (4) : and an output connected to a composite signal input of a video curve converter (7) adapted to allow the synchronized recording and/or observation of the images relating to the patient's movements and the images of the time dependency diagram of electric signals representing the activity of such organ and comprising at least one other input (7a, b, c, d) adapted to receive the electric signals representing the activity of the patient's organ and connected to the output of the receiver (3) ; and an output connected to the video recording means (8) and the display means (9).

2. A system for observing and recording the activity of at least one organ of a patient's body represented by electrically recordable phenomena, more particularly for the study and monitoring of heart diseases, comprising : video means (4) for photographing the patient's movements ; means (1, 2, 3,) for detecting and transmitting electric signals representing the activity of the organ in question and comprising in known manner : sensors (1) which are attached to the patient's body so as to receive information relating to the activity of such organ and are connected to a telemetry transmitter (2) worn by the patient and a telemetry receiver (3) ; means (6, 7) for converting and combining video signals relating to the patient's movements photographed by the photographing means (4) with images of digital information relating to the functioning of the organ in question and images of the time dependency diagram of electric signals representing the activity of such organs ; means (8) for recording such combined signals ; and means (9) for the simultaneous display of the images relating to the patient's movements, images of digital information and images of the time dependency diagram of the electric signals representing the activity of such organ, characterized in that an image cutter (28) for distributing various items of video information with a view to their display in corresponding zones of the display means (9) comprises ; a first input connected to the output of a video frequency meter (6) receiving at at least one input (6a, b, c, d) connected to the output of the receiver (3) electric signals representing the activity of such organ ; a second input connected to the output of a video curve converter (7) receiving at at least one input (7a, b, c, d) connected to the output of the receiver (3) electric signals representing the activity of such organ ; a third input connected to the output of the photographing means (4) ; a fourth input connected to the output of a video clock (5) ; and an output connected to the video recording means (8) and the video display means (9) with a view to the simultaneous recording and/or display of the images coming from the photographing means (4), the images of digital information coming from the video frequency meter (6), the images of the time dependency diagram coming from the video curve converter (7) and images coming from the video clock (5) in independent and corresponding zones of the video image.

3. A system according to one of claims 1 or 2, characterized in that the video curve converter (7) comprises an analog to digital converter (21) of the signals coming from the receiver (3), the analog to digital converter being connected to an input of a decoder-comparator (22), another input of which is connected to the output of a video coder (24) receiving at its input the video output signals of a circuit (23) for separating the signals coming from the photographing means.

4. A system according to one of claims 1 or 2, characterized in that the video frequency meter (6) comprises a mixing circuit (12) one input of which is connected to an output of a circuit (10) for separating the signals coming from the photographing means (4), another input of the mixing circuit (12) being connected to means (15, 16, 16a) for converting the output signals of the receiver (3) into video signals.

5. A system according to claim 4, characterized in that the means for converting the output signals of the receiver (3) into video signals comprise an analog to digital converter (15) whose input is connected to the receiver (3) and whose output is connected to a video coding circuit (16), the output of the latter being connected to the corresponding input of the mixing circuit (12) vin a decoding circuit (16a).

6. A system according to claim 5, characterized in that the output of the analog to digital converter (13) is connected to the recording input of an audio track of the video recording means (8), the corresponding readout output of the latter being connected to the input of the video coding circuit (16).

7. A system according to claim 3, characterized in that the input of the video curve converter (7) adapted to receive the signals of the receiver is connected to a recording input on an audio track of the video recording means (8). the corresponding output of the latter being connected to the input of the analog to digital converter (21).

8. A system according to any of the preceding claims. characterized in that it also comprises a microphone (29) connected to a transmitter (29a) worn by the patient and a receiver (29b) of the signals of the transmitter (29a) which is associated with the microphone, the receiver (29b) being connected to a recording input on an audio track of the recording means.

9. A system according to one of claims 1 to 8, characterized in that the video frequency meter (5) and/or the video curve converter (7) comprise a number of inputs (6a to 6d : 7a to 7d) which are similar to the input connected to the receiver (3) and are adapted to receive signals relating to the activity of other organs of the patient or similar organs of a number of patients and/or to environmental parameters in which the or each patient moves.

10. A system according to any of claims 1 to 9, characterized in that the photographing means (4) are formed by a camera disposed on a support in the zone of movement of the patient, and means are provided which automatically train the camera on the patient which comprise electric servomotors for moving the camera in azimuth and in position and are controlled by a device for detecting the signals of one of the sensors (1) worn by the patient.

**Patentansprüche**

1. Vorrichtung zum Beobachten und Aufzeichnen der Aktivität wenigstens eines Körperorganes eines Patienten, die durch aufzeichenbare elektrische Werte zum Ausdruck kommt, insbesondere hinsichtlich der Untersuchung und Beobachtung von Herzerkrankungen, mit Bildaufzeichnungseinrichtungen (4) zum Aufnehmen der Bewegungen des Patienten, Einrichtungen (1, 2, 3) zum Aufnehmen und Übertragen von elektrischen Signalen, die die Aktivität des betreffenden Organs wiedergeben, die in an sich bekannter Weise Fühler (1), die am Körper des Patienten so angebracht sind, daß sie Informationen bezüglich der Aktivität dieses Organes empfangen, und die mit einem Meßwertfernübertragungssender (2) verbunden sind, der vom Patienten getragen wird, und einen Meßwertfernübertragungsempfänger (3) umfassen, Einrichtungen (6, 7) zum Umwandeln und Kombinieren der Bildsignale bezüglich der Bewegungen des Patienten, die von den Bildaufzeichnungseinrichtungen (4) aufgenommen werden, mit verschlüsselten Bildinformationen bezüglich der Funktion des betreffenden Organs und mit Diagrammdarstellungen der elektrischen Signale, die die Aktivitäten dieses Organs wiedergeben, als Funktion der Zeit, Einrichtungen (8) zum Aufzeichnen dieser kombinierten Signale und Einrichtungen (9) zum simulten Anzeigen der Bilder bezüglich der Bewegungen des Patienten, der Bilder der verschlüsselten Informationen und der Diagrammdarstellungen der elektrichen Signale, die die Aktivität des besagten Organs wiedergeben, als Funktion der Zeit, dadurch gekennzeichnet, daß ein Bildfrequenzmeßgerät (6), das dazu bestimmt ist, die synchrone Aufzeichnung und/oder Beobachtung der Bilder bezüglich der Bewegungen des Patienten, die durch die Bildaufzeichnungseinrichtungen (4) aufgenommen werden, und der Bilder der verschlüsselten Informationen bezüglich der Funktion des betreffenden Organs zu ermöglichen, wenigstens einen Eingang (6a, b, c, d), der dazu bestimmt ist, die elektrischen Signale, die die Aktivität des Organs des Patienten wiedergeben, zu empfangen und der mit einem Ausgang des Empfängers (3) verbunden ist, einen weiteren Eingang, der dazu bestimmt ist, ein Bildsignal und ein Synchronsignal zu empfangen und der mit einem Bildtaktgeber (5) am Ausgang der Bildaufzeichnungseinrichtungen (4) verbunden ist, und einen Ausgang aufweist, der mit einem Eingang für das zusammengesetzte Signal eines Bildkurvenwandlers (7) verbunden ist, der dazu bestimmt ist, die synchrone Aufzeichnung und/oder Beobachtung der Bilder bezüglich der Bewegungen des Patienten und der Diagrammdarstellungen der elektrischen Signale, die die Aktivität des besagten Organs wiedergeben, als Funktion der Zeit zu ermöglichen, und der wenigstens einen weiteren Eingang (7a, b, c, d), der dazu bestimmt ist, die elektrischen Signale zu empfangen, die die Aktivität des Organs des Patienten wiedergeben, und der mit dem Ausgang des Empfängers (3) verbunden ist, und einen Ausgang aufweist, der mit den Einrichtungen (8) zum Aufzeichnen und den Bildanzeigeeinrichtungen (9) verbunden ist.

2. Vorrichtung zum Beobachten und Aufzeichnen der Aktivität wenigstens eines Körperorgans eines Patienten, die durch aufzeichenbare elektrische Werte zum Ausdruck kommt, insbesondere hinsichtlich der Untersuchung und Beobachtung von Herzkrankheiten mit Bildaufzeichnungseinrichtungen (4) zum Aufnehmen der Bewegungen des Patienten, Einrichtungen (1, 2, 3) zum Aufnehmen und Übertragen von elektrischen Signalen, die die Aktivität des betreffenden Organs wiedergeben, die in an sich bekannter Weise Fühler (1), die am Körper des Patienten so angebracht sind, daß sie Informationen bezüglich der Aktivität dieses Organs empfangen, und die mit einem Meßwertfernübertragungssender (2) verbunden sind, der vom Patienten getragen wird, und einen Meßwertfernübertragungsempfänger (3) umfassen, Einrichtungen (6, 7) zum Umwandeln und Kombinieren der Bildsignale bezüglich der Bewegungen des Patienten, die von den Bildaufzeichnungseinrichtungen (4) aufgenommen werden, mit verschlüsselten Bildinformationen bezüglich der Funktion des betreffenden Organs und mit Diagrammdarstellung der elektrischen Signale, die die Aktivität dieses Organs wiedergeben, als Funktion der Zeit, Einrichtungen (8) zum Aufzeichnen dieser kombinierten Signale und Einrichtungen (9) zum simultanen Anzeigen der Bilder bezüglich der Bewegungen des Patienten, der Bilder der verschlüsselten Informationen und der Diagrammdarstellungen der elektrischen Signale, die die Aktivität des besagten Organs wiedergeben, als Funktion der Zeit, dadurch gekennzeichnet, daß ein Bildteiler (28), der eine Trennung der verschiedenen Bildinformationen im Hinblick auf ihre Anzeige in entsprechenden Bereichen der Einrichtungen (9) zum Anzeigen bewirkt, einen ersten Eingang, der mit einem Ausgang eines Frequenzmeßgerätes (6) verbunden ist, das an wenigstens einem Eingang (6a, b, c, d), der mit dem Ausgang des Empfängers (3) verbunden ist, elektrische Signale empfängt, die die Aktivität des besagten Organs wiedergeben, einen zweiten Eingang, der mit dem Ausgang eines Bildkurvenwandlers (7) verbunden ist, der an wenigstens einem Eingang (7a, b, c, d), der mit dem Ausgang des Empfängers (3) verbunden ist, elektrische Signale empfängt, die die Aktivität des besagten Organs wiedergeben, einen dritten Eingang, der mit dem Ausgang der Bildaufzeichnungseinrichtungen (4) verbunden ist, einen vierten Eingang, der mit dem Ausgang eines Bildtaktgebers (5) verbunden ist, und einen Ausgang aufweist, der mit den Einrichtungen (8) zum Aufzeichnen und den Bildanzeigeeinrichtungen (9) verbunden ist, um gleichzeitig die Bilder, die von den Bildaufzeichnungseinrichtungen (4) kommen, die Bilder der verschlüsselten Informationen, die vom Bildfrequenzmeßgerät (6) kommen, die Bilder der Diagrammdarstellungen als Funktion der Zeit, die vom Bildkurvenwandler (7) kommen, und die Bilder vom Bildtaktgeber (5) an unabhängigen und dem Videobild entsprechenden Zonen aufzuzeichnen und/oder anzuzeigen.

3. Vorrichtung nach einem der Ansprüche 1 und 2, dadurch gekennzeichnet, daß der Bildkurvenwandler (7) einen Analogdigitalwandler (21) für die Signale vom Empfänger (3) aufweist, welcher Analogdigitalwandler mit einem Eingang eines Dekodierers und Komparators (22) verbunden ist, dessen anderer Eingang mit einem Ausgang eines Bildkodierers (24) verbunden ist, der an seinem Eingang die Ausgangsbildsignale einer Trennstufe (23) für die Signale von den Aufzeichnungseinrichtungen empfängt.

4. Vorrichtung nach einem der Ansprüche 1 und 2, dadurch gekennzeichnet, daß das Bildfrequenzmeßgerät (6) eine Mischschaltung (12) aufweist, deren einer Eingang mit einem Ausgang einer Trennschaltung (10) für die Signale von den Aufzeichnungseinrichtungen (4) verbunden ist, wobei der andere Eingang dieser Mischschaltung (12) mit Einrichtungen (15, 16, 16a) zum Umwandeln der Ausgangssignale des Empfängers (3) in Bildsignale verbunden ist.

5. Vorrichtung nach Anspruch 4, dadurch gekennzeichnet, daß die Einrichtungen zum Umwandeln der Ausgangssignale des Empfängers (3) in Bildsignale einen Analogdigitalwandler (15) aufweisen, dessen Eingang mit dem Empfänger (3) verbunden ist und dessen Ausgang mit einer Bildkodierschaltung (16) verbunden ist, deren Ausgang mit einem entsprechenden Eingang der Mischschaltung (12) über eine Dekodierschaltung (16a) verbunden ist.

6. Vorrichtung nach Anspruch 5, dadurch gekennzeichnet, daß der Ausgang des Analogdigitalwandlers (13) mit einem Aufzeichnungseeingang einer Tonspur der Einrichtungen zur Bildaufzeichnung (8) verbunden ist, wobei der Leseausgang letzterer mit einem Eingang der Bildkodierschaltung (16) verbunden ist.

7. Vorrichtung nach Anspruch 3, dadurch gekennzeichnet, daß der Eingang des Bildkurvenwandlers (7), der dazu bestimmt ist, die Signale des Empfängers zu empfangen, mit einem Aufzeichnungseingang einer Tonspur der Einrichtungen zur Bildaufzeichnung (8) verbunden ist, wobei der entsprechende Ausgang letzterer mit einem Eingang des Analogdigitalwandlers (21) verbunden ist.

8. Vorrichtung nach einem der vorhergehenden Ansprüche, dadurch gekennzeichnet, daß sie weiterhin ein Mikrophon (29), das mit einem Sender (29a) verbunden ist, der vom Patienten getragen wird, und einen Empfänger (29b) für die Signale des Senders (29a) aufweist, der dem Mikrophon zugeordnet ist, wobei der Empfänger (29b) mit einem Aufzeichnungseingang einer Tonspur der Aufzeichnungseinrichtungen verbunden ist.

9. Vorrichtung nach einem der Ansprüche 1 bis 8, dadurch gekennzeichnet, daß das Bildfrequenzmeßgerät (6) und/oder der Bildkurvenwandler (7) mehrere Eingänge (6a bis 6d ; 7a bis 7d) aufweist/aufweisen, die den mit dem Empfänger (3) verbundenen Eingang ähnlich sind und dazu bestimmt sind, Signale bezüglich der Aktivität anderer Organe des Patienten oder ähnlicher Organe mehrerer Patienten und/oder der Parameter der Umgebung zu empfangen, in der sich der Patient oder die Patienten bewegen.

10. Vorrichtung nach einem der Ansprüche 1 bis 9, dadurch gekennzeichnet, daß die Bildaufzeichnungseinrichtungen (4) aus einer Kamera bestehen, die auf einem Stativ im Bereich der Bewegung des Patienten angebracht ist, und daß Einrichtungen zum automatischen Ausrichten der Kamera auf den Patienten vorgesehen sind, die elektrische Servomotoren zur Azimutal- und Höhenverstellung der Kamera umfassen, die von einer Detektoreinrichtung für die Signale eines der vom Patienten getragenen Fühler (1) gesteuert werden.

FIG. 1

1

Sortie
Horloge
5

10

13

11

12

14

17

Vers
Convertisseur
7

Sortie
Récepteur
3

15

16

16a

## FIG.2

15

16

Sortie 7

8

## FIG.3

FIG.4

FIG.5

FIG.5A